# EUROPÄISCHE PATENTSCHRIFT

(11) **EP 0 545 126 B1**
(45) Veröffentlichungstag und Bekanntmachung des Hinweises auf die Patenterteilung: **31.01.1996**
(21) Anmeldenummer: 92119513.7
(22) Anmeldetag: 14.11.1992
(51) Int. Cl.: C08F 251/00, C08F 283/06, A61L 15/00

(54) **Hydrophile quellfähige Pfropfpolymerisate**
Hydrophilic water swellable graft copolymers
Copolymères greffés hydrophiles gonflables à l'eau

(30) Priorität: 30.11.1991 DE 4139613
(43) Veröffentlichungstag der Anmeldung: 09.06.1993
(73) Patentinhaber: HOECHST AKTIENGESELLSCHAFT, D-65926 Frankfurt am Main (DE)
(72) Erfinder: Kussmaul, Ulrich, Dr., W-6367 Karben 1 (DE); Stüven, Uwe, W-6232 Bad Soden (DE); Engelhardt, Friedrich, Dr., W-6000 Frankfurt 60 (DE); Mayer, Manfred, Dr., W-6272 Niedernhausen (DE); Riegel, Ulrich, W-6000 Frankfurt 60 (DE)

(56) Entgegenhaltungen:
- EP-A- 0 283 090
- EP-A- 0 316 792
- EP-A- 0 388 254
- EP-A- 0 400 283
- EP-A- 0 455 965
- US-E-RE32957 (R.T. ELIAS)

## Beschreibung

Die vorliegende Erfindung betrifft hydrophile quellfähige Pfropfpolymerisate, deren Herstellung und Verwendung als Absorptionsmittel für Wasser und wäßrige Lösungen, zum Beispiel in Hygieneartikeln, zur Bodenverbesserung oder als Filtrationshilfsmittel.

Quellbare Polymere, die wäßrige Lösungen absorbieren, werden für die Herstellung von Tampons, Windeln, Damenbinden und anderen Hygieneartikeln sowie als wasserzurückhaltende Mittel im landwirtschaftlichen Gartenbau verwendet.

Zu bekannten Absorptionsharzen dieses Typs gehören vernetzte Carboxymethylcellulose, teilweise vernetztes Polyalkylenoxid, Hydrolysate von Stärke-Acrylnitril-Pfropfcopolymeren, teilweise vernetzte Polyacrylsäuresalze oder auch Pfropfpolymerisate wie sie in der EP-A 400 283 beschrieben sind.

Diese bekannten Polymerisate zeigen durchweg Nachteile, insbesondere bei der Absorption wäßriger Elektrolytlösung sowie Blut und Urin.

Bei, hohem Absorptionsvermögen werden nach dem derzeitigen Stand der Technik zu geringe Gelfestigkeiten der gequollenen Polymerpartikel erreicht. Es bilden sich klebrige Massen, welche die Saugfähigkeit der damit hergestellten Produkte verschlechtern.

Es ist bekannt, daß durch Erhöhung der Vernetzungsdichte die Gelfestigkeit sowie die Geschwindigkeit der Flüssigkeitsaufnahme erhöht werden kann, dadurch jedoch gleichzeitig die Absorptionskapazität herabgesetzt wird. Diese Vorgehensweise ist insofern unerwünscht als die Absorptionskapazität die wichtigste Eigenschaft des Polymeren ist.

Die bekannten Polymerisate neigen außerdem auf heißen Stahloberflächen, beispielsweise auf Walzentrocknerflächen, zum Ankleben, was die Trocknung erheblich erschwert. Insbesondere können solche Polymerisate nicht mit einer ausreichend hohen Geschwindigkeit getrocknet werden.

Aufgabe der vorliegenden Erfindung ist es, modifizierte Polymere, die wäßrige Lösungen absorbieren, bereitzustellen, welche eine hohe Absorptionsrate aufweisen, dabei im gequollenen Zustand nicht klebende Hydrogelpartikel hoher Gelfestigkeit bilden und die an heißen Stahloberflächen nicht zum Ankleben neigen.

Die vorliegende Erfindung betrifft hydrophile, quellbare Pfropfpolymerisate, die in statistischer Verteilung zu 0,5 bis 50 Gew.% aus Resten der allgemeinen Formel I 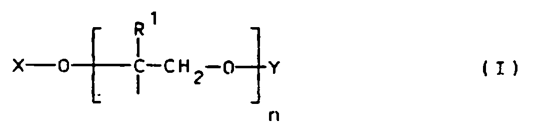 und Resten der allgemeinen Formel II 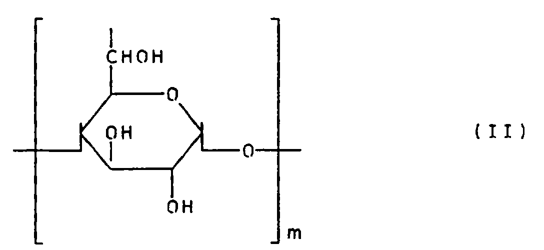 zu 49 bis 99 Gew.% aus eine saure Gruppe enthaltenden Resten der allgemeinen Formel III 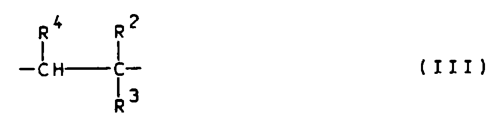 und zu 0,1 bis 2 Gew.% aus Resten eines Vernetzers, die aus Monomeren mit mindestens zwei olefinisch ungesättigten Doppelbindungen hervorgegangen sind, bestehen, wobei X (C₁-C₂₂)-Alkyl, Aryl, Aralkyl oder Y, 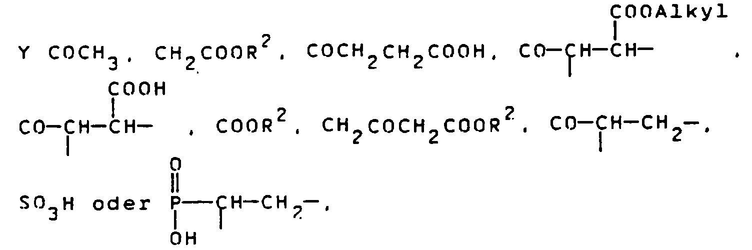 n und m unabhängig voneinander 2 bis 300,
R¹ Wasserstoff oder Methyl,
R unabhängig voneinander Wasserstoff, Methyl oder Ethyl,
R³ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel 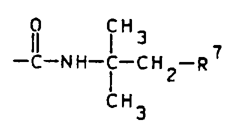 worin R⁷ für die Sulfonylgruppe oder die Phosphonylgruppe steht,
R⁴ Wasserstoff, Methyl, Ethyl oder die Carboxylgruppe bedeuten.

Bevorzugte erfindungsgemäße Produkte bestehen zu 0,5 bis 20 Gew.% aus Resten der allgemeinen Formeln I und II, 79 bis 99 Gew.% aus Resten der allgemeinen Formel III und 0,1 bis 1,8 Gew.% aus vernetzenden Strukturen, die aus Monomeren mit mindestens zwei. olefinisch ungesättigten Doppelbindungen hervorgegangen sind.

Besonders bevorzugte erfindungsgemäße Produkte bestehen zu 1 bis 15,5 Gew.% aus Resten der allgemeinen Formeln I und II, 83 bis 98,5 Gew.% aus Resten der allgemeinen Formel III und 0,3 bis 1,5 Gew.% aus vernetzenden Strukturen, die aus Monomeren mit mindestens zwei olefinisch ungesättigten Doppelbindungen hervorgegangen sind.

Das Gewichtsverhältnis der Reste der allgemeinen Formeln I und II beträgt bevorzugt 1 : 99 bis 99 : 1. Das Gewichtsverhältnis der Reste der allgemeinen Formeln I und II beträgt besonders bevorzugt 50 : 50, wenn der Anteil der Summe der Reste der allgemeinen Formeln I und II am Gesamtpolymerisat weniger als 10 Gew.% beträgt und 10 : 90 bis 25 : 75, wenn der Anteil der Summe der Reste der allgemeinen Formeln I und II am Gesamtpolymerisat 10 bis 50 Gew.% beträgt.

In den erfindungsgemäßen Pfropfcopolymerisaten können die Reste der allgemeinen Formel I alle exakt die gleiche Struktur haben, sie können sich jedoch auch hinsichtlich des Restes R¹ und/oder der Zahl n voneinander unterscheiden. So können sich bezüglich R¹ Wasserstoff und Methyl in statistischer Weise abwechseln, es können aber auch größere Polymerabschnitte aufeinander folgen, in denen R¹ jeweils nur Wasserstoff oder nur Methyl bedeutet.

Für X stehendes Aryl hat bevorzugt 3 bis 8 Kohlenstoffatome und bedeutet besonders bevorzugt Phenyl, tert.-Butyl-phenyl und Nonylphenyl. Für X stehendes Aralkyl hat bevorzugt 3 bis 8 Kohlenstoffatome im Aryl- und 1 bis 22 Kohlenstoffatome im Alkylrest.

Die Alkylgruppe des für Y stehenden Restes 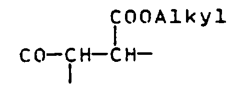 hat bevorzugt 1 bis 22 Kohlenstoffatome.
Y bedeutet bevorzugt
COCH₃, CH₂COOR, COCH₂CH₂COOH, COOR, CH₂COCH₂COOR₂ und

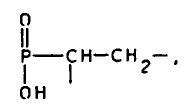

Die Reste der allgemeinen Formel II leiten sich von Stärke bzw. Abbauprodukten von Stärke ab.

In den Resten der allgemeinen Formel III bedeutet R bevorzugt Wasserstoff oder Methyl. R³ steht bevorzugt für die Carboxylgruppe, die Sulfonylgruppe oder die Phosphonylgruppe. Besonders bevorzugt ist die Carboxylgruppe. R⁴ bedeutet bevorzugt Wasserstoff.

Die genannten vernetzenden Strukturen können sich von allen geeigneten Monomeren mit mindestens zwei olefinisch ungesättigten Doppelbindungen ableiten.

Geeignete Monomere sind beispielsweise Verbindungen, die mindestens zwei Alkenylgruppen, zum Beispiel Vinyl oder Allyl, oder mindestens zwei Alkenoylgruppen, zum Beispiel Acrylat oder Methacrylat, enthalten.

Bevorzugt leiten sich die vernetzenden Strukturen von Monomeren ab, die 2, 3 oder 4 ethylenisch ungesättigte Doppelbindungen enthalten.

Besonders bevorzugt leiten sich die vernetzenden Strukturen von Trimethylolpropantriacrylat, Tetraallyloxyethan oder Methylenbisacrylamid ab.

Weitere vernetzende Strukturen können erhalten werden durch Zusatz von mehrfunktionellen Epoxiden, wie z.B. Ethylenglykoldiglycidylether oder cycloaliphatischem Diepoxid.

Ganz besonders bevorzugte erfindungsgemäße Pfropfpolymerisate sind solche, in denen mehrere der oben genannten bevorzugten oder besonders bevorzugten Merkmale enthalten sind.

Die erfindungsgemäßen Pfropfpolymerisate können durch bekannte Polymerisationsverfahren hergestellt werden. Bevorzugt ist die Polymerisation in wäßriger Lösung nach dem Verfahren der sogenannten Gelpolymerisation. Dabei werden 15 - 50 %ige wäßrige Lösungen der Comonomeren mit bekannten geeigneten Katalysatorsystemen ohne mechanische Durchmischung unter Ausnutzung des Trommsdorff-Norrish-Effektes (Bios Final Rep. 363.22; Makromol. Chem. 1, 169 (1947)) polymerisiert.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert. Butylhydroperoxid, Methyl-ethyl-keton-peroxid, Cumol-hydroperoxid, Azoverbindungen wie Azo-di-iso-butyro-nitril sowie anorganische Peroxiverbindungen wie (NH₄)₂S₂O₈ oder K₂S₂O₈ oder H₂O₂ gegebenenfalls in Kombination mit Reduktionsmitteln wie Natriumhydrogensulfit, und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren, enthalten, wie z.B. Mannichaddukte aus Sulfinsäure, Aldehyden und Aminoverbindungen, wie sie in der deutschen Patentschrift 13 01 566 beschrieben sind. Pro 100 g Gesamtmonomeren werden in der Regel 0,03 bis 2 g des Polymerisationsinitiators eingesetzt.

Durch mehrstündiges Nachheizen der Polymerisatgele im Temperaturbereich von 50 - 130°C, vorzugsweise 70 - 100°C, können die Qualitätseigenschaften der Polymerisate noch verbessert werden.

Die auf diesem Wege hergestellten, in Form wäßriger Gallerten vorliegenden erfindungsgemäßen Copolymerisate können nach mechanischer Zerkleinerung mit geeigneten Apparaten durch bekannte Trocknungsverfahren in fester Form erhalten werden und zum Einsatz gelangen.

Zweckmäßigerweise werden somit erfindungsgemäße Pfropfpolymerisate erhalten, wenn 0,5 bis 50 Gew.%, vorzugsweise 0,5 bis 20, insbesondere 1 bis 15,5 Gew.%, einer Mischung aus einer Polyalkylenoxidverbindung der allgemeinen Formel Ia 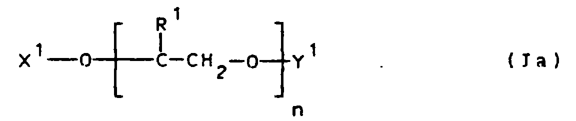 oder gegebenenfalls ein Alkali-, Ammonium- oder Aminsalz davon und einer Verbindung der allgemeinen Formel IIa 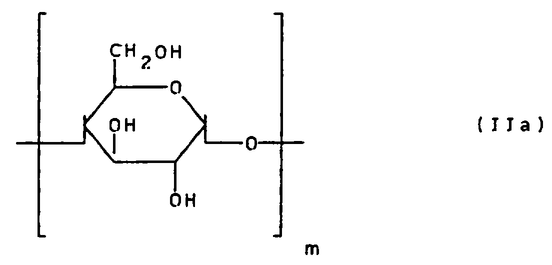 49 bis 99 Gew.%, vorzugsweise 79 bis 99, insbesondere 83 bis 98,5 Gew.%, einer ungesättigten Säure der allgemeinen Formel IIIa 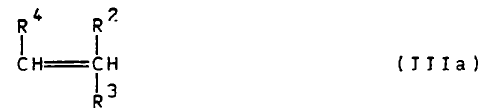 oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei
X¹ (C₁-C₂₂)-Alkyl, Aryl, Aralkyl oder Y,
Y¹ COCH₃, CH₂COOR, COCH₂CH₂COOH, CO-CH=CH-COOAlkyl,
CO-CH=CH-COOH, COOR, CH₂COCH₂COOR, CO-CH=CH₂, SO₃H oder 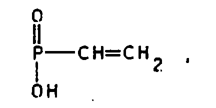 und die Reste R¹ bis R⁴ und die Zahlen n und m die obengenannten Bedeutungen haben, unter den Bedingungen der Gelpolymerisation umgesetzt werden.

Die Polyalkylenoxidverbindungen der allgemeinen Formel Ia können durch bekannte Umsetzungsreaktionen von Verbindungen mit reaktiven Gruppen, wie Anhydride, Säurechloride, Halogencarbonsäuren oder deren Estern oder Halogensulfonsäuren und Polyalkylenoxiden erhalten werden.

Bevorzugte Polyalkylenoxide sind Polypropylen- und Polyethylenoxide, Co- oder Blockcopolymere aus Ethylenoxid und Propylenoxid, Oxethylate, Oxpropylate oder Oxethyloxpropylate von aliphatischen C₁ bis C₂₂ Alkylalkoholen, Phenol, tert.Butylphenol oder Nonylphenol.

Bevorzugte Reagenzien zum Endverschluß der Polymerkette sind Chloracetessigsäure und deren Ester, Chlorameisensäure und deren Ester, Vinylphosphonsäuremono- und Dichlorid, Bernsteinsäureanhydrid, Essigsäureanhydrid, Monochloressigsäure.

Bevorzugte Verbindungen der allgemeinen Formel IIa sind Stärken. Es können dabei alle nativen und/oder modifizierten Stärken verschiedener Herkunft verwendet werden. Stärken aus Wurzeln und Knollen verschiedener Pflanzen, wie zum Beispiel Kartoffeln, Maranta oder Maniok, können ebenso eingesetzt werden wie solche aus Getreidesamen, wie zum Beispiel Mais, Weizen, Reis und Gerste, solchen aus Früchten, wie zum Beispiel Kastanien, Erbsen und Bohnen oder solchen aus Mark, wie zum Beispiel dem der Sagopalme.

Besonders bevorzugte Verbindungen der allgemeinen Formel IIa sind Mais- und Kartoffelstärke.

Die Monomeren der Formel IIIa sind bekannte Verbindungen, wie zum Beispiel Acrylsäure, Methacrylsäure, Vinylsulfonsäure, Maleinsäure, Fumarsäure, Crotonsäure, 2-Acrylamido-2-methyl-propansulfonsäure, 2-Acrylamido-2-methylpropanphosphonsäure und Vinylphosphonsäure sowie deren Halbester.

Die als Vernetzer eingesetzten polyolefinischen Monomere sind gängige Produkte. Beispiele sind Bisacrylamidoessigsäure, Trimethylolpropantriacrylat, Tetraallyloxyethan und Methylenbisacrylamid.

Besonders bevorzugte erfindungsgemäße Polymerisate sind solche, die im Anschluß an die oben beschriebene Herstellung mechanisch zerkleinert und durch Aufbringen von carboxylgruppenreaktiven Verbindungen auf die Oberfläche der Polymerpartikel modifiziert werden.

Geeignete carboxylgruppenreaktive Verbindungen sind beispielsweise mehrwertige Alkohole, Polyalkohole, Polyepoxide, (Poly)-glycidylether, Phosphonsäurediglycidylester, Polyamine, Polyoxazoline, Polyaziridine, Alkoxysilanamine, Alkoxysilanepoxide, Polyamidoamine, polyquarternäre Substanzen und mehrwertige Metallkationen.

Bevorzugte carboxylgruppenreaktive Verbindungen sind Glycidylether mehrwertiger Alkohole, wie z.B. Monoethylenglykoldiglycidylether, Polyethylenglykoldiglycidylether, insbesondere Nonaethylenglykoldiglycidylether, Monopropylenglykoldiglycidylether und Polypropylenglykoldiglycidylether; Glycidylphosphonsäureester der allgemeinen Formel IV 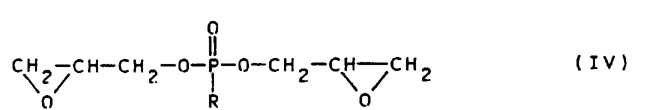 worin R Alkyl, Alkenyl oder Aryl, die gegebenenfalls substituiert sein können, bedeutet;
polymere Verbindungen, wie z.B. Polyamine oder Polyamidoamine, welche in bekannter Weise durch Kondensation aliphatischer Dicarbonsäuren mit Di- oder Polyaminen, wie z.B. Diethylentriamin, hergestellt werden und auch quarternisiert sein können;
Alkoxysilylverbindungen wie z.B. Aminoalkylalkoxysilane, insbesondere 3-Aminopropyltriethoxysilan, Trimethoxysilylpropyldiethylentriam.in, N-Aminoethylaminopropyltrimethoxysilan und Aminoethylaminomethylphenethyltrimethoxysilan sowie Glycidylalkoxysilane, insbesondere 3-Glycidyloxypropyltrimethoxysilan; sowie hochreaktive Kieselsäuresole.

In der allgemeinen Formel IV bedeutet R bevorzugt (C₁-C₁₈)-Alkyl; (C₃-C₈)-Cycloalkyl; eine Gruppe der allgemeinen Formel V 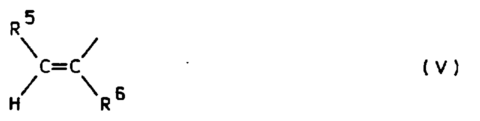 wobei R⁵ und R⁶ unabhängig voneinander für Wasserstoff oder (C₁-C₄)-Alkyl stehen; oder eine Gruppe der allgemeinen Formel VI 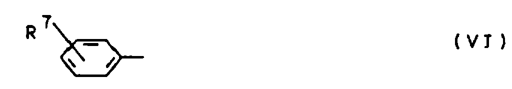 wobei R⁷ für Wasserstoff, Halogen oder (C₁-C₄)-Alkyl steht.

Die genannten carboxylgruppenreaktiven Verbindungen können auch in Mischungen untereinander oder stufenweise nacheinander angewendet werden.

Das Aufbringen der carboxylgruppenhaltigen Verbindungen auf die Polymerpartikel geschieht bevorzugt durch Mischen der Komponenten in einem Mischaggregat.

Geeignete Mischaggregate sind beispielsweise Patterson-Kelly-Mischer, DRAIS-Turbolenzmischer, Lödige-Mischer, Schneckenmischer, Tellermischer und Wirbelschichtmischer.

Das Verfahren wird bevorzugt im Temperaturbereich zwischen 0 und 200°C, bevorzugt zwischen 10 und 80°C, ausgeführt. Es ist dabei bevorzugt, die Polymerpartikel vor Ausführung des Verfahrens auf eine Temperatur von 40 bis 100°C vorzuwärmen. Tn einer bevorzugten Ausführungsform werden die Komponenten bei einer Temperatur zwischen 20 und 60°C in einem üblichen, heizbaren Mischaggregat gemischt und dann zur Beschleunigung der Reaktion im oberflächennahen Bereich auf eine Temperatur zwischen 80 und 200°C, bevorzugt 80 bis 1500C, erhitzt. In einer weiteren bevorzugten Ausführungsform erfolgt dieser Temperaturbehandlungsschritt in einem nachgeschalteten Trockner über einen Zeitraum von 10 Minuten bis 6 Stunden, wobei sowohl Spaltprodukte, welche bei der Reaktion entstehen können, sowie eventuell vorher zugesetzte Lösungsmittelanteile entfernt werden können.

Die carboxylgruppenreaktiven Verbindungen können sowohl in Substanz als auch in Form von Lösungen eingesetzt werden.

Bevorzugte Lösungsmittel sind Wasser, sowie Alkohole, Ester, Ketone, Ether und Kohlenwasserstoffe sowie Mischungen dieser Komponenten mit Kochpunkten bis zu 200°C, bevorzugt bis 150°C.

Die carboxylgruppenreaktiven Verbindungen werden dabei bevorzugt in Mengen von 0,01 bis 10 Gew.%, besonders bevorzugt 0,05 bis 3 Gew.%, bezogen auf die zu modifizierenden Polymerpartikel, eingesetzt.

Die erfindungsgemäßen Pfropfpolymerisate eignen sich hervorragend als Absorptionsmittel für Wasser und wäßrige Lösungen, so daß sie vorteilhaft als wasserzurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Von besonderem Vorteil ist, daß die erfindungsgemäßen Pfropfpolymerisate nicht zum Ankleben an heißen Stahloberflächen, wie z.B. Walzentrocknerflächen, neigen und somit eine hohe Trocknungsgeschwindigkeit gewährleistet ist.

In den nachstehenden Beispielen werden die folgenden Verbindungen der allgemeinen Formel Ia verwendet:

### A:

In 312 g eines Blockcopolymeren aus 1,03 mol Propylenoxid und 0,91 mol Ethylenoxid mit der OH-Zahl 36, werden unter Rühren bei. Raumtemperatur 20,0 g Bernsteinsäureanhydrid eingetragen und diese lt.ischung unter Rühren auf 80°C erhitzt. Das Bernsteinsäureanhydrid löst sich dabei unter schwach exothermer Reaktion auf; es entsteht eine klare farblose Lösung.

### B:

In einem Vierhalskolben mit Azeotropaufsatz und Stickstoffeinleitung werden 1010 g (0,495 mol) Polypropylenglykol 2020 in 500 ml Toluol gelöst und entwässert, d.h. es wird 3 h azeotrop destilliert, wobei 43 g Wasser abgeschieden werden. Bei 105 - 110°C werden im Laufe von 30 min 112,5 g (1,1 mol) Essigsäureanhydrid zugetropft, 2 h bei 105 - 11o°C nachgerührt. Unter Wasserstrahlvakuum werden Toluol, Essigsäure und überschüssiges Essigsäureanhydrid abdestilliert. Als Rückstand verbleiben 1020 g eines farblosen Öls.

### C:

4621 g (1.0 mol) eines Polyglykolethers auf Basis Nonylphenol mit 100 Ethylenoxideinheiten werden geschmolzen.

Bei 90 - 100°C werden 102 g (1,0 mol) Acetanhydrid zugetropft, 30 min gerührt und dann unter Wasserstrahlvakuum die entstandene Essigsäure abdestilliert. Es entsteht eine farblose Lösung, die bei Raumtemperatur zu einem festen Wachs erstarrt.

### D:

In einem Reaktionskolben werden 345 g eines Blockpolymeren aus 1,6 mol Propylenoxid und 0,2 mol Ethylenoxid mit der OH-Zahl 65 in 350 ml Essigsäureethylester gelöst, 40,5 g Triethylamin zugesetzt und langsam 37,8 g Monochloressigsäure zugegeben. Es wird 1 h nachgerührt, das Triethylaminhydrochlorid abgesaugt und das Lösungsmittel unter Wasserstrahlvakuum abdestilliert. Als Rückstand verbleiben 368 g eines farblosen Öls.

### E:

Umsetzung analog A mit einem Copolymeren aus 0,35 mol Propylenoxid und 1,82 mol Ethylenoxid mit der OH-Zahl 17.

### F:

Umsetzung analog A mit einem Copolymeren aus 1,6 mol Propylenoxid und 0,2 mol Ethylenoxid mit der OH-Zahl 65.

### G:

Umsetzung analog D mit Nonylphenoloxethylat mit 30 Ethyleneinheiten und Chloracetessigsäureethylester.

### H:

Umsetzung analog D mit Nonylphenoloxethylat mit 30 Ethyleneinheiten und Chlorameisensäure.

### T:

Umsetzung analog D mit einem Copolymeren aus 1,03 mol Propylenoxid und 0,91 mol Ethylenoxid mit der OH-Zahl 36 und Vinylphosphonsäuremonochlorid.

### K:

Umsetzung analog D mit Polypropylenglykol 2020 und Monochloressigsäure.

### L:

Umsetzung analog C mit tert. Butylphenoloxethylat mit 80 Ethylenoxideinheiten und Acetanhydrid.

### M:

Umsetzung analog D mit Phenoloxethylat mit 15 Propylenoxideinheiten und Vinylphosphonsäuredichlorid.

### N:

Umsetzung analog B mit Polyethylenglykol 300 (1,0 Mol) und Maleinsäureanhydrid (1,98 Mol).

### Beispiel 1:

In einen durch geschäumtes Kunststoffmaterial gut isolierten Polyethyleneimer mit einem Fassungsvermögen von 10 l werden 4430 g entsalztes Wasser vorgelegt, 1493 g Natriumbicarbonat darin dispergiert und langsam 1910 g Acrylsäure so zudosiert, daß ein Überschäumen der Reaktionslösung vermieden wird, wobei sich diese auf eine Temperatur von ca. 12 - 10°C abkühlt. Es werden nun 40 g des Produktes gemäß A (siehe oben), 490 g einer aufgekochten und ebenfalls auf 10°C abgekühlten Stärkelösung aus 45 g handelsüblicher Maisstärke und 445 g E-Wasser, 20 g Trimethylolpropantriacrylat, gelöst in 20 g eines Polyglykolethers auf Basis eines synthetischen C₁₂-C₁₅-Oxoalkohols mit 13 Ethylenoxid-Einheiten und 10 g eines Natrium-Diisooctylsulfosuccinates (REWOPOL V 2133 der Firma REWO, Steinau) zugegeben. Bei einer Temperatur von 10 - 12°C werden die Initiatoren, ein Redoxsystem, bestehend aus 2,2 g 2,2'-Azobisamidinopropan-Dihydrochlorid, gelöst in 20 g Wasser, 4,4 g Kaliumperoxidisulfat, gelöst in 170 g Wasser, und 6 g Natriumpyrosulfit, gelöst in 120 g Wasser, nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht. Dieses wird anschließend mechanisch zerkleinert, bei. Temperaturen über 80°C getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

### Beispiel 2:

In einem 10 Liter-Kunststoffeimer werden 4419 g Eis und 1894 g Acrylsäure vorgelegt und langsam 1573 g NaOH 50%ig zudosiert, anschließend 100 g des Produktes gemäß A) (siehe oben), 275 g einer aufgekochten und auf 20°C abgekühlten Stärkelösung aus 25 g handelsüblicher Kartoffelstärke und 250 g enthärtetes Wasser und 6 g Methylenbisacrylamid dispergiert in 100 g Wasser zugegeben. Die Reaktionslösung wird auf 20°C eingestellt und anschliessend mit den Initiatoren, ein Redoxsystem bestehend aus 2,2 g 2,2'-Azobisamidinopropan-dihydrochlorid, gelöst in 20 g Wasser, 6 g Kaliumperoxidisulfat, gelöst in 170 g Wasser, und 0,15 g Ascorbinsäure, gelöst in 120 g Wasser, versetzt und ohne Rühren stehen gelassen. Das durch Polymerisation entstehende Gel wird anschließend mechanisch zerkleinert, bei Temperaturen über 80°C getrocknet und gemahlen.

### Beispiel 3:

Unter adiabatischen Bedingungen werden in einem 5 l zylindrischen Weithalsreaktionskolben 2900 g E-Wasser vorgelegt, 1250 g einer handelsüblichen kaltwasserlöslichen Stärke, 15 g des Produktes gemäß N), 1250 g Acrylsäure sowie 0,625 g Tetraallyloxyethan darin gelöst und auf 20°C eingestellt. Es wird Stickstoff in die Monomerlösung eingeleitet (ca. 2 l/Min.), um den Sauerstoffgehalt zu erniedrigen. Bei einem Gehalt von ca. 0,8 ppm 02 werden 34 g einer 4 %igen wässrigen Lösung von 2,2'-Azobis(Amidinopropan)dihydrochlorid zugegeben, nach weiterem N₂-Einleiten und einem O₂-Gehalt von ca. 0,08 ppm werden
17 g einer 0,75 %igen H₂O₂-Lösung zugegeben und schließlich bei einem O₂-Gehalt von ca. 0,01 ppm werden 4,5 g einer 0,15 %igen Ascorbinsäurelösung zugegeben. Durch einsetzende Polymerisation, in deren Verlauf die Temperatur bis auf ca. 90°C ansteigt, entsteht ein festes Gel, das anschließend mechanisch zerkleinert wird. 1000 g des zerkleinerten Gels werden mit 346 g Natronlauge 27 %ig versetzt (Neutralisationsgrad der Acrylsäure = 70 Mol-%), dreimal durchgeknetet, und anschließend bei Temperaturen über 100°C in dünner Schicht getrocknet, gemahlen und gegebenenfalls gesiebt.

Wietere Beispiele zur Herstellung erfindungsgemäßer Pfropfpolymerisate gemäß der hier beschriebenen Beispiele 1 und 2 sind in folgender Tabelle zusammengefaßt. Die Mengenangaben bedeuten Gewichts-% bezogen auf Gesamtmonomeranteil. Als Verbindung der allgemeinen Formel IIa wurde in jedem Fall Maisstärke verwendet.

Folgende Abkürzungen werden benutzt:
- AS:: Acrylsäure
- MAS:: Methacrylsäure
- CTS:: Crotonsäure
- VPS:: Vinylphosphonsäure
- VPE:: Vinylphosphonsäurehalbester
- AMP:: 2-Acrylamido-2-methyl-propansulfonsäure
- AMPP:: 2-Acrylamido-2-methyl-propanphosphonsäure
- TMPTA:: Trimethylolpropantriacetat
- TAE:: Tetraallyloxyethan
- MBA:: Methylenbisacrylamid

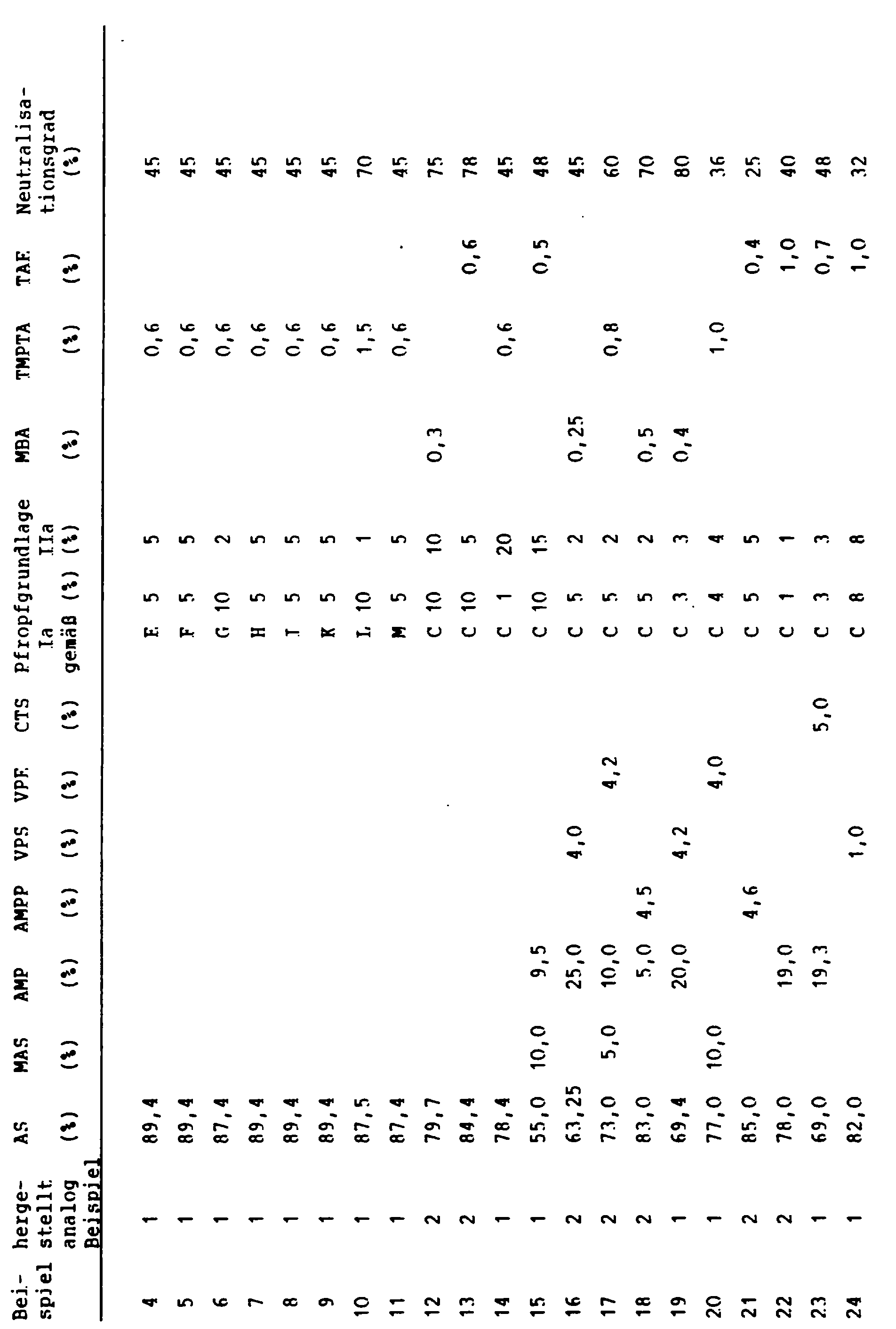

## Patentansprüche

1. Hydrophile, quellbare Pfropfpolymerisate, die in statistischer Verteilung zu 0,5 bis 50 Gew.% aus Resten der allgemeinen Formel I 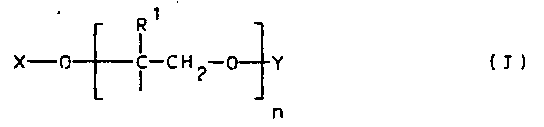 und Resten der allgemeinen Formel II 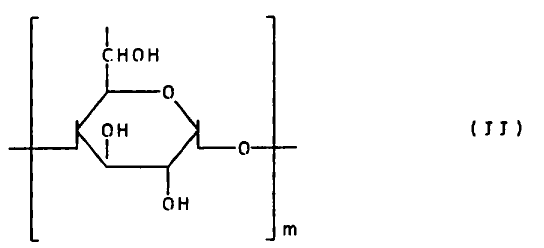 zu 49 bis 99 Gew.% aus einer sauren Gruppe enthaltenden Resten der allgemeinen Formel III 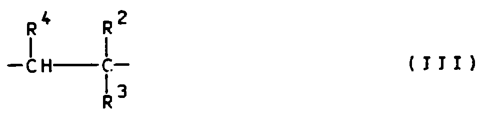 und zu 0,1 bis 2 Gew.% aus Resten eines Vernetzers, die aus Monomeren mit mindestens zwei olefinisch ungesättigten Doppelbindungen hervorgegangen sind, bestehen, wobei X (C₁-C₂₂)-Alkyl, Aryl, Aralkyl oder Y, 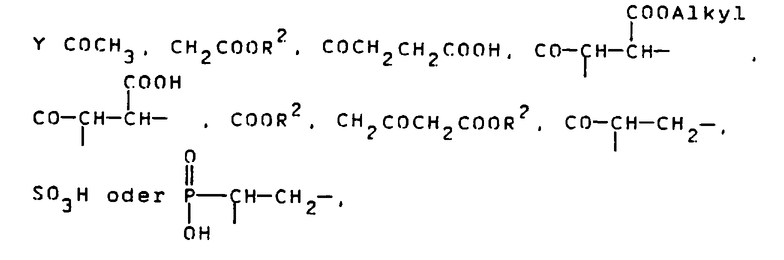 n und m unabhängig voneinander 2 bis 300,
R¹ Wasserstoff oder Methyl,
R unabhängig voneinander Wasserstoff, Methyl oder Ethyl,
R³ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel 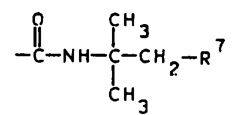 worin R⁷ für die Sulfonylgruppe oder die Phosphonylgruppe steht,
R⁴ Wasserstoff, Methyl, Ethyl oder die Carboxylgruppe bedeuten.

2. Pfropfpolymerisate gemäß Anspruch 1, dadurch gekennzeichnet, daß sie in statistischer Verteilung zu 1 bis 15,5 Gew.% aus Resten der allgemeinen Formeln I und II, zu 83 bis 98,5 Gew.% aus Resten der allgemeinen Formel III und zu 0,3 bis 1,5 Gew.% aus vernetzenden Strukturen bestehen.

3. Pfropfpolymerisate gemäß Ansprüchen 1 und/oder 2, dadurch gekennzeichnet, daß sich die Reste der allgemeinen Formel I hinsichtlich des Restes R¹ und/oder der Zahl n voneinander unterscheiden.

4. Pfropfpolymerisate gemäß einem oder mehreren der Ansprüche 1 bis 3, dadurch gekennzeichnet, daß in den Resten der allgemeinen Formel III R Wasserstoff oder Methyl, R³ die Carboxylgruppe, die Sulfonylgruppe oder die Phosphonylgruppe und R⁴ Wasserstoff bedeuten.

5. Pfropfpolymerisate gemäß einem oder mehreren der Ansprüche 1 bis 4, dadurch gekennzeichnet, daß sich die vernetzenden Strukturen von Monomeren mit mindestens zwei Alkenylgruppen oder mindestens zwei Alkenoylgruppen, insbesondere von Trimethylolpropantriacrylat, Tetraallyloxyethan oder Methylenbisacrylamid ableiten.

6. Pfropfpolymerisate gemäß einem oder mehreren der Ansprüche 1 bis 5, dadurch gekennzeichnet, daß sie durch Aufbringen von carboxylgruppenreaktiven Verbindungen auf ihre Oberflächen modifiziert sind.

7. Pfropfpolymerisate gemäß Anspruch 6, dadurch gekennzeichnet, daß als carboxylgruppenreaktive Verbindungen Glycidylether mehrwertiger Alkohole, wie z.B. Monoethylenglykoldiglycidylether, Polyethylenglykoldiglycidylether, insbesondere Nonaethylenglykoldiglycidylether, Monopropylenglykoldiglycidylether und Polypropylenglykoldiglycidylether; Glycidylphosphonsäureester der allgemeinen Formel IV 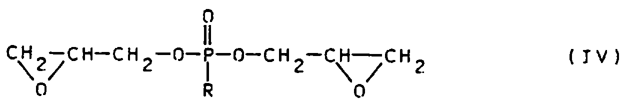 worin R Alkyl, Alkenyl oder Aryl, die gegebenenfalls substituiert sein können, bedeutet;
polymere Verbindungen, wie z.B. Polyamine oder Polyamidoamine, welche in bekannter Weise durch Kondensation aliphatischer Dicarbonsäuren mit Di- oder Polyaminen, wie z.B. Diethylentriamin, hergestellt werden und auch quarternisiert sein können;
Alkoxysilylverbindungen wie z.B. Aminoalkylalkoxysilane, insbesondere 3-Aminopropyltriethoxysilan, Trimethoxysilylpropyldiethylentriamin, N-Aminoethylaminopropyltrimethoxysilan und Aminoethylaminomethylphenethyltrimethoxysilan sowie Glycidylalkoxysilane, insbesondere 3-Glycidyloxypropyltrimethoxysilan; oder hochreaktive Kieselsäuresole eingesetzt werden.

8. Verfahren zur Herstellung der in den Ansprüchen 1 bis 7 beanspruchten Pfropfpolymerisate, dadurch gekennzeichnet, daß 0,5 bis 50 Gew.%, vorzugsweise 0,5 bis 20, insbesondere 1 bis 15,5 Gew.%, einer Mischung aus einer Polyalkylenoxidverbindung der allgemeinen Formel Ia 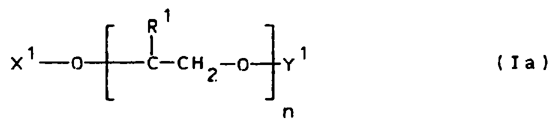 oder gegebenenfalls ein Alkali-, Ammonium- oder Aminsalz davon und einer Verbindung der allgemeinen Formel IIa 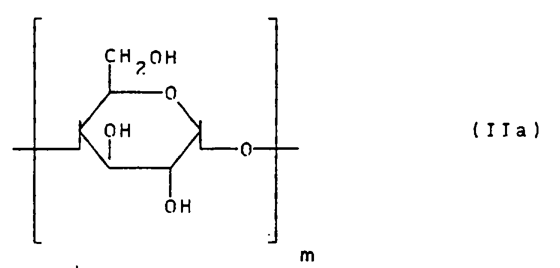 49 bis 99 Gew.%, vorzugsweise 79 bis 99, insbesondere 83 bis 98,5 Gew.%, einer ungesättigten Säure der allgemeinen Formel IIIa 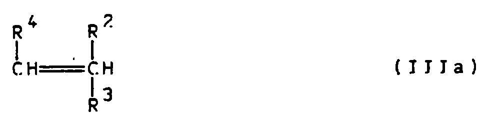 oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei
X¹ (C₁-C₂₂)-Alkyl, Aryl, Aralkyl oder Y,
Y¹ COOH, CH₂COOR, COH₂CH₂COOH, CO-CH=CH-COOAlkyl,
CO-CH=CH-COOH, COOR, CH₂COCH₂COOR, CO-CH=CH₂, SO₃H oder 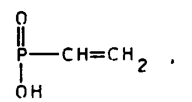 und R¹ bis R⁴ sowie n und m wie im Anspruch 1 definiert sind, unter den Bedingungen der Gelpolymerisation umgesetzt werden und im Anschluß daran gegebenenfalls das Produkt mechanisch zerkleinert und mit carboxylgruppenreaktiven Verbindungen in einem Mischaggregat gemischt wird.

9. Verfahren gemäß Anspruch 8, dadurch gekennzeichnet, daß als Verbindung der allgemeinen Formel IIa Stärke, insbesondere Mais- oder Kartoffelstärke eingesetzt wird.

10. Verwendung der in den Ansprüchen 1 bis 7 beanspruchten Pfropfpolymerisate als Absorptionsmittel für Wasser und wäßrige Lösungen.

## Claims

1. Hydrophilic, swellable graft polymers which comprise, in a random distribution, from 0.5 to 50 % by weight of radicals of the general formula I 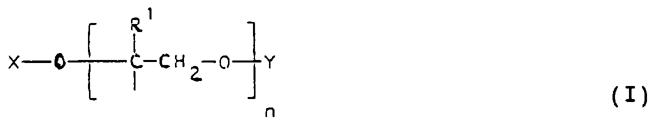 and radicals of the general formula II 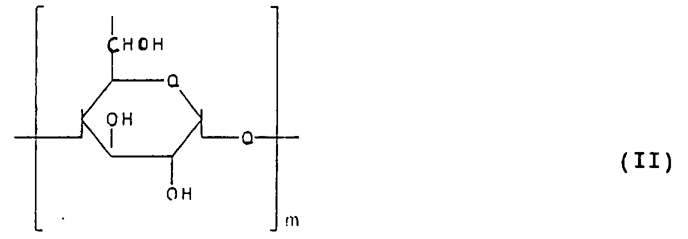 from 49 to 99 % by weight of radicals of the general formula III 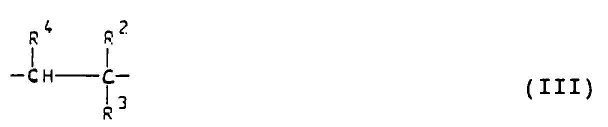 containing an acid group, and
from 0.1 to 2 % by weight of radicals of a crosslinking agent which are derived from monomers containing at least two olefinically unsaturated double bonds, where
X is (C₁-C₂₂)-alkyl, aryl, aralkyl or Y,
Y is 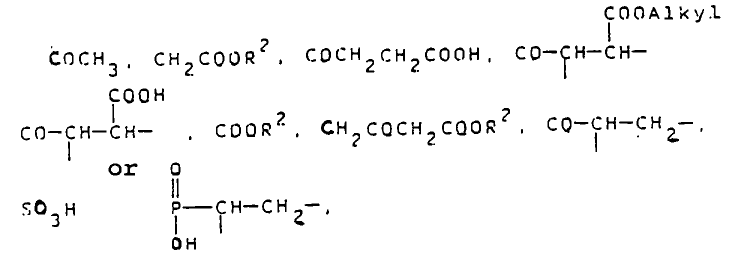 n and m, independently of one another, are 2 to 300,
R¹ is hydrogen or methyl,
the radicals R, independently of one another, are hydrogen, methyl or ethyl,
R³ is the carboxyl group, the sulphonyl group, the phosphonyl group, which is optionally esterified by means of alkanol having l to 4 carbon atoms, or a group of the formula 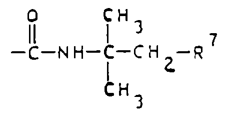 in which R⁷ is the sulphonyl group or the phosphonyl group, and R⁴ is hydrogen, methyl, ethyl or the carboxyl group.

2. Graft polymers according to Claim 1, characterised in that they comprise, in a random distribution, from l to 15.5 % by weight of radicals of the general formulae I and II, from 83 to 98.5 % by weight of radicals of the general formula III and from 0.3 to 1.5 % by weight of crosslinking structures.

3. Graft polymers according to Claims l and/or 2, characterised in that the radicals of the general formula I differ from one another with respect to the radical R¹ and/or the number n.

4. Graft polymers according to one or more of Claims 1 to 3, characterised in that, in the radicals of the general formula III, R is hydrogen or methyl, R³ is the carboxyl group, the sulphonyl group or the phosphonyl group, and R⁴ is hydrogen.

5. Graft polymers according to one or more of Claims 1 to 4, characterised in that the crosslinking structures are derived from monomers containing at least two alkenyl groups or at least two alkenoyl groups, in particular from trimethylolpropane triacrylate, tetraallyloxyethane or methylenebisacrylamide.

6. Graft polymers according to one or more of Claims 1 to 5, characterised in that they have been modified by application of carboxyl-reactive compounds to their surfaces.

7. Graft polymers according to Claim 6, characterised in that the carboxyl-reactive compounds employed are glycidyl ethers of polyhydric alcohols, such as, for example, monoethylene glycol diglycidyl ether, polyethylene glycol diglycidyl ether, in particular nonaethylene glycol diglycidyl ether, monopropylene glycol diglycidyl ether and polypropylene glycol diglycidyl ether; glycidyl phosphonates of the general formula IV 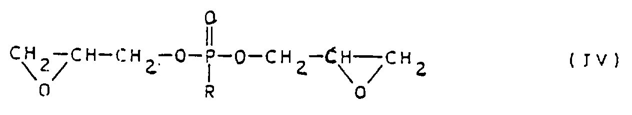 in which R is alkyl, alkenyl or aryl, each of which is optionally substituted;
polymeric compounds, such as, for example, polyamines or polyamidoamines, which are prepared in a known manner by condensation of aliphatic dicarboxylic acids with diamines or polyamines, such as, for example, diethylenetriamine, and may also be quaternised; alkoxysilyl compounds, such as, for example, aminoalkylalkoxysilanes, in particular 3-aminopropyltriethoxysilane, trimethoxysilylpropyldiethylenetriamine, N-aminoethylaminopropyltrimethoxysilane and aminoethylaminomethylphenethyltrimethoxysilane, and glycidylalkoxysilanes, in particular 3-glycidyloxypropyltrimethoxysilane; or highly reactive silicic acid sols.

8. Process for the preparation of the graft polymers claimed in Claims 1 to 7, characterised in that from 0.5 to 50 % by weight, preferably from 0.5 to 20 % by weight, in particular from 1 to 15.5 % by weight, of a mixture of a polyalkylene oxide compound of the general formula Ia 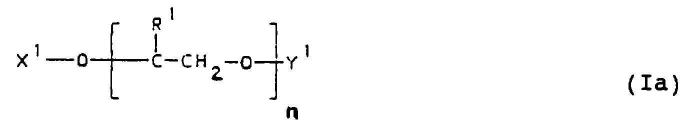 or, if desired, an alkali metal salt, ammonium salt or amine salt thereof, and a compound of the general formula IIa 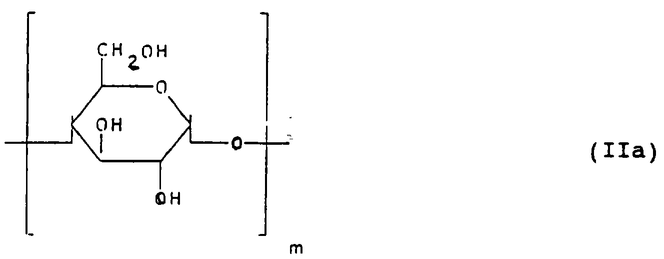 from 49 to 99 % by weight, preferably from 79 to 99 % by weight, in particular from 83 to 98.5 % by weight, of an unsaturated acid of the general formula IIIa 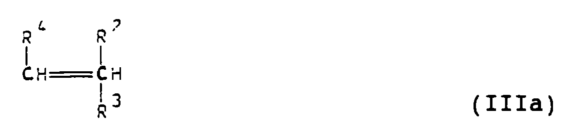 or an alkali metal salt, ammonium salt or amine salt thereof, and from 0.1 to 2 % by weight, preferably from 0.1 to 1.8 % by weight, in particular from 0.3 to 1.5 % by weight, of a monomer containing at least two olefinically unsaturated double bonds, where
X¹ is (C₁-C₂₂)-alkyl, aryl, aralkyl or Y,
Y¹ is COCH₃, CH₂COOR, COCH₂CH₂COOH, CO-CH=CH-COOalkyl,
CO-CH=CH-COOH, COOR, CH₂COCH₂COOR, CO-CH=CH₂, SO₃H or 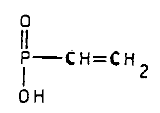 and R¹ to R⁴ and n and m are as defined in Claim 1, are reacted under the conditions of gel polymerisation, and, if desired, the product is subsequently comminuted mechanically and mixed with carboxyl-reactive compounds in a mixer.

9. Process according to Claim 8, characterised in that the compound of the general formula IIa is starch, in particular maize starch or potato starch.

10. Use of the graft polymers claimed in Claims 1 to 7 as absorbents for water and aqueous solutions.

## Revendications

1. Polymères greffés gonflables, hydrophiles, qui, selon une répartition statistique, sont constitués de 0,5 à 50 % en poids de radicaux de formule générale I 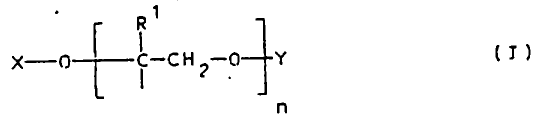 et de radicaux de formule générale II 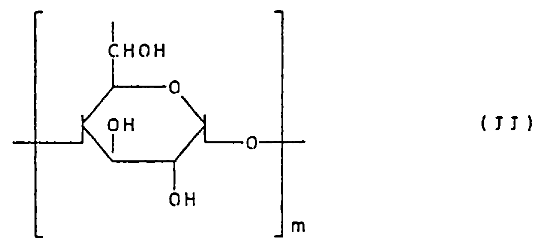 de 49 à 99 % en poids de radicaux contenant un groupe acide, de formule générale III 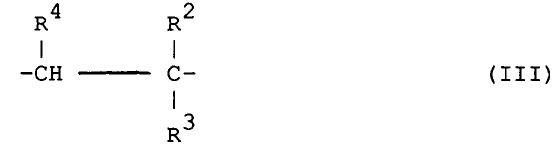 et de 0,1 à 2 % en poids d'un résidu d'un agent de réticulation, qui est obtenu à partir de monomères ayant au moins deux doubles liaisons à insaturation oléfinique, où :
X est un radical alkyle en C₁-C₂₂, aryle, aralkyle ou Y,
Y est COCH₃, CH₂COOR, COCH₂CH₂COOH, 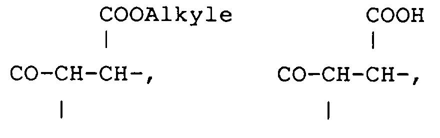 COOR, CH₂COCH₂COOR, 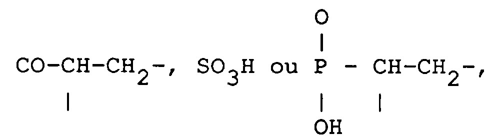
n et m, indépendamment l'un de l'autre, valent chacun de 2 à 300,
R¹ est un hydrogène ou le radical méthyle,
les radicaux R, indépendamment les uns des autres, sont chacun un hydrogène ou un radical méthyle ou éthyle,
R³ est le groupe carboxyle, le groupe sulfonyle, le groupe phosphonyle, qui éventuellement peut être estérifié par un alcanol ayant de 1 à 4 atomes de carbone, ou encore un groupe de formule 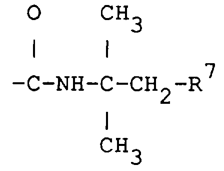 dans laquelle R⁷ représente le groupe sulfonyle ou le groupe phosphonyle,
R⁴ est un hydrogène ou le radical méthyle, éthyle, ou le groupe carboxyle.

2. Polymères greffés selon la revendication 1, caractérisés en ce qu'ils sont constitués, en réparti-tion statistique, de 1 à 15,5 % en poids de radicaux de formules générales I et II, de 83 à 98,5 % en poids de radicaux de formule générale III et de 0,3 à 1,5 % en poids de structures réticulables.

3. Polymères greffés selon les revendications 1 et/ou 2, caractérisés en ce que les radicaux de formule générale I se distinguent les uns des autres pour ce qui est du radical R¹ et/ou de l'indice n.

4. Polymères greffés selon l'une ou plusieurs des revendications 1 à 3, caractérisés en ce que, dans les radicaux de formule générale III, R est un hydrogène ou le radical méthyle, R³ est le groupe carboxyle, le groupe sulfonyle ou le groupe phosphonyle, et R⁴ est un hydrogène.

5. Polymères greffés selon l'une ou plusieurs des revendications 1 à 4, caractérisés en ce que les structures réticulables dérivent de monomères ayant au moins deux groupes alcényle ou au moins deux groupes alcénoyle, en particulier de triacrylate de triméthylolpropane, de tétraallyloxyéthane ou de méthylènebisacrylamide.

6. Polymères greffés selon l'une ou plusieurs des revendications 1 à 5, caractérisés en ce qu'ils sont modifiés par application, sur leur surface, de composés réactifs vis-à-vis des groupes carboxyle.

7. Polymères greffés selon la revendication 6, caractérisés en ce qu'on utilise comme composés réactifs vis-à-vis des groupes carboxyle les éthers glycidyliques de polyalcool tels par exemple que l'éther diglycidylique du monoéthylèneglycol, l'éther diglycidylique du polyéthylèneglycol, en particulier l'éther diglycidylique du nonaéthylèneglycol, l'éther diglycidylique du monopropylèneglycol et l'éther diglycidylique du polypropylèneglycol ; les glycidylphosphonates de formule générale IV 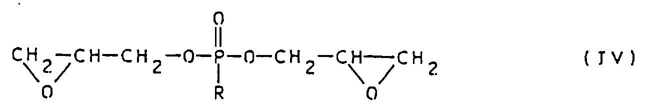 dans laquelle R est un radical alkyle, alcényle ou aryle, pouvant être éventuellement substitué ;
les composés polymères, tels par exemple que les polyamines ou polyamidoamines, que l'on peut préparer d'une manière connue par condensation d'acides dicarboxyliques aliphatiques avec des diamines ou des polyamines telles par exemple que la diéthylènetriamine, et que l'on peut aussi quaterniser ;
les composés alcoxysilylés tels par exemple que les aminoalkylalcoxysilanes, en particulier le 3-aminopropyltriéthoxysilane, la triméthoxysilylpropyldiéthylènetriamine, le N-aminoéthylaminopropyltriméthoxysilane et l'aminoéthylaminométhylphénéthyltriméthoxysilane, ainsi que les glycidylalcoxysilanes, en particulier le 3-glycidyloxypropyltriméthoxysilane ; ainsi que les sols de silice à haute réactivité.

8. Procédé pour préparer les polymères greffés revendiqués dans les revendications 1 à 7, caractérisé en ce que l'on fait réagir dans les conditions d'une polymérisation en gel une quantité de 0,5 à 50 % en poids, de préférence de 0,5 à 20 et en particulier de 1 à 15,5 % en poids, d'un mélange d'un poly(oxyde d'alkylène) de formule générale Ia 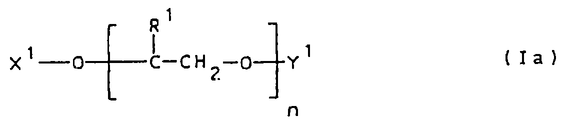 ou éventuellement d'un sel d'un métal alcalin, d'ammonium ou d'amine de ce dernier, et d'un composé de formule générale IIa 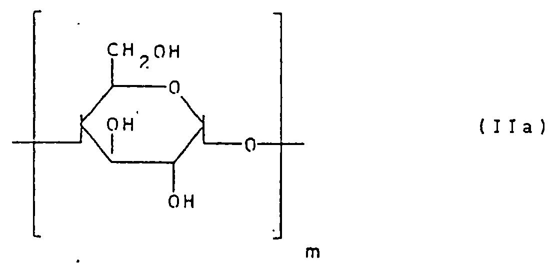 de 49 à 99 % en poids, de préférence de 79 à 99 et en particulier de 83 à 98,5 % en poids d'un acide insaturé de formule générale IIIa 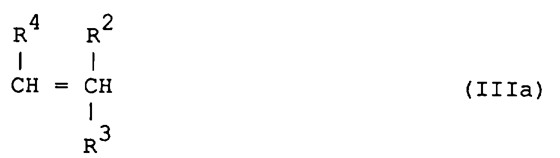 ou d'un sel d'un métal alcalin, d'ammonium ou d'amine de ce dernier, et de 0,1 à 2 % en poids, de préférence 0,1 à 1,8 et en particulier de 0,3 à 1,5 % en poids d'un monomère ayant au moins deux doubles liaisons à insaturation oléfinique, où
X¹ est un radical alkyle en C₁-C₂₂, aryle, aralkyle ou Y,
Y¹ est COCH₃, CH₂COOR, COCH₂CH₂COOH,
CO-CH=CH-COOAlkyle, CO-CH=CH-COOH, COOR,
CH₂COCH₂COOR, CO-CH=CH₂, SO₃H ou 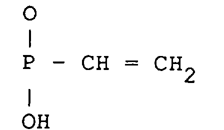
et les radicaux R¹ à R⁴ et les indices n et m sont tels que définis dans la revendication 1, puis, on sou-met éventuellement le produit à un broyage mécanique, et, dans un mélangeur, on le mélange à des composés réactifs vis-à-vis des groupes carboxyle.

9. Procédé selon la revendication 8, caractérisé en ce qu'on utilise comme composé de formule générale IIa de l'amidon, notamment de l'amidon de maïs ou de pomme de terre.

10. Utilisation des polymères greffés selon les revendications 1 à 7 comme absorbant pour l'eau et les solutions aqueuses.
